(19) Europäisches Patentamt
European Patent Office
Office européen des brevets

(11) EP 4 613 275 A1

(12) EUROPEAN PATENT APPLICATION
published in accordance with Art. 153(4) EPC

(43) Date of publication:
**10.09.2025 Bulletin 2025/37**

(21) Application number: **23884745.3**

(22) Date of filing: **27.10.2023**

(51) International Patent Classification (IPC):
***A61K 31/7068*** *(2006.01)* ***A61K 31/7072*** *(2006.01)*
***A61K 31/706*** *(2006.01)* ***A61K 31/7076*** *(2006.01)*
***A61P 31/20*** *(2006.01)*

(52) Cooperative Patent Classification (CPC):
**A61K 31/706; A61K 31/7068; A61K 31/7072; A61K 31/7076; A61P 31/20**

(86) International application number:
**PCT/CN2023/127028**

(87) International publication number:
**WO 2024/093813 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.11.2022 CN 202211367238**

(71) Applicant: **Henan Genuine Biotech Co., Ltd.**
**Pingdingshan, Henan 467000 (CN)**

(72) Inventors:
- **WANG, Zhaoyang**
  **Pingdingshan, Henan 467000 (CN)**
- **LI, Pan**
  **Pingdingshan, Henan 467000 (CN)**
- **JIA, Limin**
  **Pingdingshan, Henan 467000 (CN)**

(74) Representative: **Bandpay & Greuter**
**11, rue Christophe Colomb**
**75008 Paris (FR)**

# (54) PHARMACEUTICAL USE OF NUCLEOSIDE COMPOUND

(57) Provided is pharmaceutical use of a nucleoside compound, specifically use of a compound represented by formula (I) or a pharmaceutically acceptable salt thereof in the preparation of a drug for preventing or treating an infectious disease of orthopoxvirus. The compound represented by formula (I) has a significant inhibitory effect on a vaccinia virus, a monkeypox virus, a cowpox virus, and a smallpox virus.

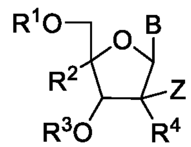

(I)

EP 4 613 275 A1

## Description

### TECHNICAL FIELD

The present disclosure relates to use of a nucleoside compound in the preparation of a drug for preventing or treating an orthopoxvirus infectious disease.

### BACKGROUND

Most viruses of the genus *Orthopoxvirus* in the *Poxviridae* family cause systemic exanthema.

Smallpox virus is the pathogen of smallpox in humans. The initial symptoms after infection with smallpox virus include a high fever, fatigue, a headache, a rapid heartbeat, and back pain, followed by characteristic smallpox rash prominently distributed on the face, arms, and legs. In the early stage of rash, there would also be reddish lumpy areas that accompany the rash. The lesions begin to suppurate and scab over after a few days, then slowly develop into scabies, and subsequently fall off slowly.

Symptoms in humans infected with monkeypox virus are similar to those observed in patients infected with viruses such as smallpox virus in the past. This virus can be transmitted from animals to humans through direct close contact or spread from person to person. The main routes of infection include blood and body fluids. On May 20, 2022, there were more than 100 confirmed and suspected cases of monkeypox in Europe. On May 29, 2022, the World Health Organization (WHO) issued the disease information circular and assessed the global public health risk of monkeypox as "moderate". On June 24, 2022, the number of confirmed cases of monkeypox worldwide was over 3,200. On July 23, 2022, the WHO classified the monkeypox outbreak as "Public Health Emergency of International Concern". As of September 2022, the data released by the Centers for Disease Control and Prevention of the United States showed that there were more than 25,000 confirmed cases in the United States. The monkeypox virus is a serious hazard with rapid onset, strong infectivity and a high lethality rate.

Cowpox virus is a virus that may cause mild lesions in cattle. Humans, if infected with this virus, will experience mild discomfort and build resistance to the cowpox virus.

Weak in pathogenicity as it is, vaccinia virus is sufficiently potent to induce the body to produce antibodies against diseases. It is bound up with the smallpox virus in serology and immunology and is used as an antigen in the smallpox vaccine.

### SUMMARY

Compound 1 (Azvudine) has already been approved for marketing as a drug for treatment of AIDS and COVID-19, and its safety is guaranteed.

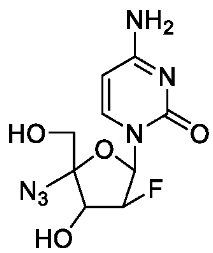

1

The present inventors have tested the activity of Compound 1 to inhibit monkeypox virus, smallpox virus, cowpox virus, and vaccinia virus. The results show that Compound 1 has a significant inhibitory effect on all of these viruses.

In view of this, in one aspect of the present disclosure, there is provided use of a compound represented by formula (I) or a pharmaceutically acceptable salt thereof in the preparation of a drug for preventing or treating an orthopoxvirus infectious disease. In another aspect of the present disclosure, there is provided a method for preventing or treating an orthopoxvirus infectious disease, comprising: administering, to a subject in need thereof, a therapeutically or prophylactically effective amount of a compound of formula (I) or a pharmaceutically acceptable salt thereof. In another aspect of the present disclosure, there is provided a pharmaceutical composition, comprising a compound represented by formula (I) or a pharmaceutically acceptable salt thereof and an optional excipient, wherein the pharmaceutical composition is used for preventing or treating an orthopoxvirus infectious disease.

The structure of formula (I) is as follows:

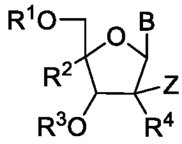

(I)

in formula (I),

$R^1$ is any group that enables the $OR^1$ group to metabolize *in vivo* to release a hydroxyl group or form an O-phosphate group; $OR^1$ is preferably an ester group, $R^1$ is preferably H, $R^5$-CO- or

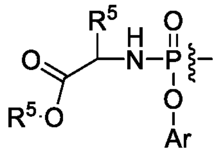

,

wherein Ar is phenyl, substituted phenyl, naphthyl or substituted naphthyl, and the substituent is selected from $C_{1-6}$ alkyl, F, Cl, Br, I, CN, $N_3$, OH, $NH_2$, $OR^5$, and $NHR^5$

$R^2$ is selected from: H, azido, $C_1$-$C_6$ alkyl (such as methyl or ethyl), $C_1$-$C_6$ alkoxy (such as methoxy or ethoxy), $C_2$-$C_6$ alkynyl (such as ethynyl), $C_2$-$C_6$ alkenyl (such as vinyl), and halogenated $C_1$-$C_6$ alkyl (such as 2-chloroethyl, 2-fluoroethyl or trifluoroethyl);

$R^3$ is selected from H, optionally substituted R-CO-, optionally substituted R-O(C=O)-, and optionally substituted RNH-CO-, wherein R is selected from $C_1$-$C_6$ alkyl (such as methyl, ethyl, propyl, isopropyl, butyl, isobutyl, pentyl or isopentyl); wherein the substituent is selected from $C_1$-$C_6$ alkyl, halogen (such as F or Cl), CN, $N_3$, and $OR^5$;

$R^4$ is selected from H, OH, halogen (such as F), $C_1$-$C_6$ alkyl (such as methyl or ethyl), and $C_1$-$C_6$ alkoxy (such as methoxy or ethoxy);

B is selected from:

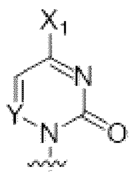

and

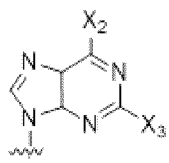

,

wherein $X_1$ is selected from -OH, $-NH_2$, $R^5$CONH-, $R^5$COO-, and $R^5$O (C=O)NH-;

$X_2$ is selected from -OH, -SH, $-NH_2$, $R^5$COO-, $R^5$COS-, $R^5CONH_2$-, and $R^5$O (C=O)NH-;

$X_3$ is selected from H, F, -OH, and $-NH_2$;

Y is selected from CH and N;

Z is H, -OH or F;

$R^5$ is selected from H, $C_1$-$C_6$ alkyl (such as methyl, ethyl, propyl or isopropyl), $C_2$-$C_6$ alkynyl (such as ethynyl), $C_2$-$C_6$ alkenyl (such as vinyl), halogenated $C_1$-$C_6$ alkyl (such as 2-chloroethyl, 2-fluoroethyl or trifluoroethyl), phenyl optionally substituted with $C_{1-6}$ alkyl, $C_{1-6}$ alkoxy, CN, $N_3$, OH, $NH_2$ or halogen (such as F, Cl, Br or I), and naphthyl optionally substituted with $C_{1-6}$ alkyl, $C_{1-6}$ alkoxy, CN, $N_3$, OH, $NH_2$ or halogen (such as F, Cl, Br or I).

Preferably, in formula (I),

$R^1$ is H or $R^5$-CO-;

$R^2$ is azido;

$R^3$ is H or R-CO-, wherein R is $C_1$-$C_6$ alkyl;

$R^4$ is halogen;

B is:

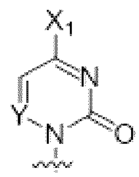

,

wherein $X_1$ is $-NH_2$;
Y is CH;
Z is H;
$R^5$ is selected from H and $C_1$-$C_6$ alkyl.

The following compounds or pharmaceutically acceptable salts thereof are preferred:

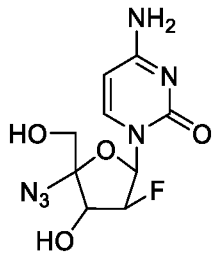
,
**1**

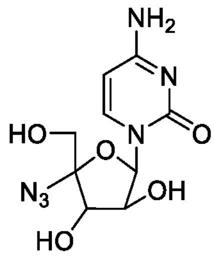
,
**2**

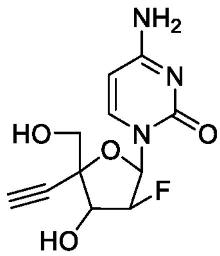
,
**3**

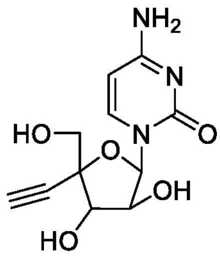
,
**4**

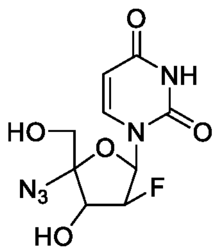
,
**5**

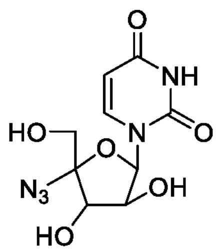
,
**6**

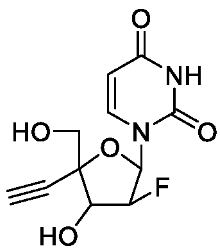
,
**7**

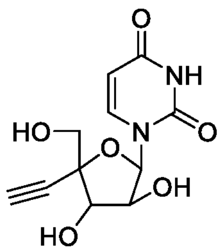

**8**

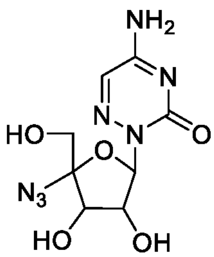
,
**9**

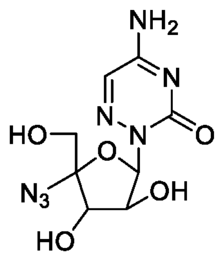
,
**10**

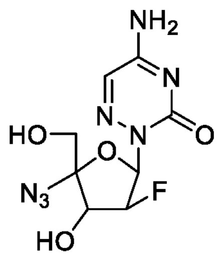
,
**11**

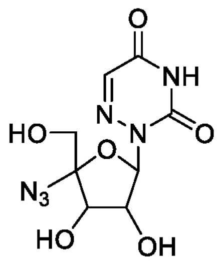

**12**

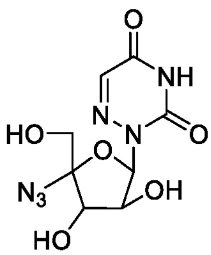
,
**13**

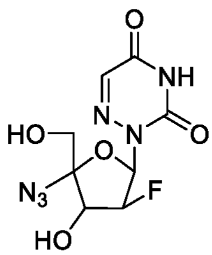
,
**14**

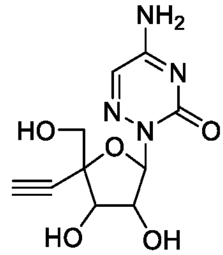
,
**15**

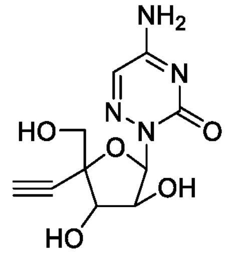

**16**

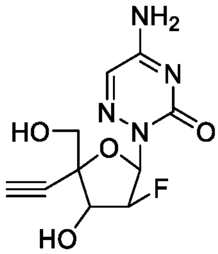
,
17

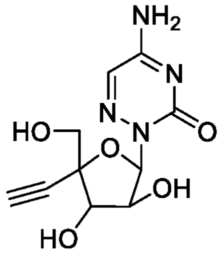
,
18

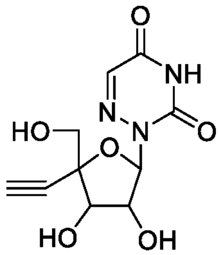
,
19

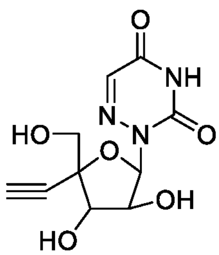

20

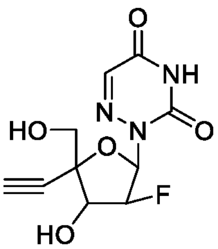
,
21

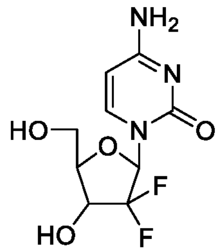
,
22

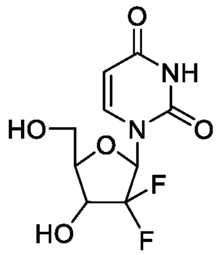
,
23

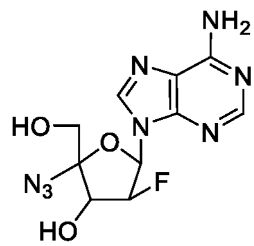
,
24

and

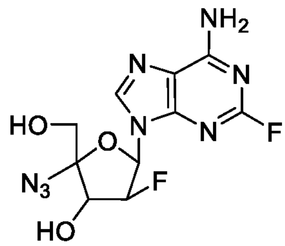
.
25

The pharmaceutically acceptable salt of the compound of formula (I) includes, but is not limited to, for example, salts formed of the compound of formula (I) with the following acids: hydrochloric acid, hydrobromic acid, sulfamic acid, sulfuric acid, phosphoric acid, nitric acid, formic acid, acetic acid, propionic acid, oxalic acid, glycolic acid, malonic acid, benzoic acid, lactic acid, gluconic acid, citric acid, tartaric acid, succinic acid, fumaric acid, maleic acid, mandelic acid, malic acid, methanesulfonic acid, ethanesulfonic acid, isethionic acid, benzenesulfonic acid, p-toluenesulfonic acid, naphthalenesulfonic acid, naphthalenedisulfonic acid, camphorsulfonic acid, ascorbyl palmitic acid, salicylic acid, sulfosalicylic acid, 2-hydroxy-3-naphthoic acid, phthalic acid, lysine, arginine, glutamic acid, glycine, serine, threonine, alanine, isoleucine, leucine, etc.

The compound of formula (I) may be commercially available or prepared by known methods.

The orthopoxvirus infectious disease used herein is a disease caused by infection with viruses of the genus *Orthopoxvirus,* including diseases caused by infection of humans or other animals, particularly diseases caused by infection of humans. The orthopoxvirus includes, for example, monkeypox virus, cowpox virus, and smallpox virus.

The effective amount of the compound of formula (I) for preventing or treating the orthopoxvirus infectious disease may be determined by a person skilled in the art based on the information provided herein. For example, the dosage for an adult may be 1 to 500 mg/day, optionally 1 to 50 mg/day, optionally 1 to 20 mg/day, optionally 1 to 10 mg/day, or optionally 5 mg/day. It can be administered in a single dose or in multiple doses.

The mode of administration may be oral or parenteral administration. It may be an immediate-release dosage form, or may be a sustained-release dosage form or a controlled-release dosage form. The specific dosage forms may be various conventional dosage forms in the art. For example, examples of the oral preparation may include tablets, hard or soft capsules, aqueous or oily suspensions, granules, emulsions, syrup or elixirs. Examples of the injection may include injections or powder injections.

## DETAILED DESCRIPTION

The specific embodiments of the present disclosure will be described in detail below. It should be appreciated that the specific embodiments described herein are merely intended to illustrate the present disclosure and not to limit the present disclosure.

### Example: Antiviral Activity Assay on Compound

### 1.1 Cell Lines and Virus Strains

Human foreskin fibroblasts HFF-1 and the virus strains of Vaccinia VP13, Monkeypox virus, Cowpox virus, and Smallpox virus were obtained from American Type Culture Collection (ATCC) and China Center for Type Culture Collection (CCTCC), respectively. The experimental medium was DMEM medium supplemented with 2% fetal bovine serum, 2 mM L-glutamine, 1% non-essential amino acids, 1% dual resistance, and 1% sodium pyruvate.

### 1.2 Compound

The test compound was a solid powder of Compound 1 provided by Henan Genuine Biotech Co., Ltd., and was formulated into a 20 mM stock solution with 100% DMSO. Control compound ST-246 (MedChemExpress, Cat. No.: HY-14805) was provided by WuXi AppTec. The compound was tested at 8 concentrations each for 3-fold serial dilutions and triplicate wells. The starting test concentration of the test compound was 10 μM. The maximum test concentration was listed in Table 1.

**Table 1. Information of Compounds**

| Compound | Molecular Weight | Maximum Test Concentration | Remarks |
|---|---|---|---|
| Compound 1 | 286.22 | 10 μM | Test compound |
| ST-246 | 376.33 | 1 μM and 100 μM* | Control compound |
| *Note: The maximum test concentrations of ST-246 for antiviral activity and cytotoxicity were 1 μM and 100 μM, respectively. | | | |

### 1.3 **Main Reagent and Instrument**

(1) Main instrument: Microplate Reader (Molecule Devices, model: SpectraMax340PC384).
(2) Main reagent: Cell Counting Kit CCK-8 (Shanghai Life iLab Biotech Co., LTD, Cat. No.: AC11L057).

### 2. **Experimental Method**

ST-246 was a control compound for activity assay. The compound was tested at 8 concentrations each for triplicate wells.

**Table 2. Experimental Method for Antiviral Activity**

| Virus | Cells | Compound Treatment Period (day)/ Experiment | Positive Control |
|---|---|---|---|
| Vaccinia/VP13 | HFF-1 | 4/CPE | ST-246 |
| Monkeypox virus | HFF-1 | 4/CPE | ST-246 |
| Cowpox virus | HFF-1 | 4/CPE | ST-246 |
| Smallpox virus | HFF-1 | 4/CPE | ST-246 |

Day 1: HFF-1 cells were inoculated into a 96-well test plate at a density of 6,000 cells in 100 μL per well, and cultured overnight in an incubator at 5% $CO_2$, 37°C.

Day 2: 50 μL of serially diluted compound (8 concentrations, 3-fold dilution, triplicate wells) and 50 μL of viruses (at an inoculation volume of MOI=0.1) were added to each well, respectively. Cell control (cells, no compound treatment

and virus infection), virus control (cells infected with virus, no compound treatment) and medium control (medium only) were set up. The total volume of cell medium was 200 μL per well. The final concentrations of DMSO in the media were 0.5%, respectively. The cells were cultured in an incubator at 5% $CO_2$, 37°C for 4 days.

Day 6: Cell viability was detected by Cell Counting Kit CCK8. The antiviral activity and cytotoxicity of the test samples were expressed by the inhibition rate (%) of the cytopathic effect caused by the virus at different concentrations and the cell viability (%), respectively. They were calculated by the following formulae:

$$\textit{Inhibition Rate (\%)} = \textit{(test well reading - virus control average)/ (cell control average - virus control average)} \times 100$$

$$\textit{Cell Viability (\%)} = \textit{(test well reading - medium control average)/ (cell control average - medium control average)} \times 100$$

$EC_{50}$ and $CC_{50}$ values were calculated by the Prism software (version 7), and the curve of inhibition was fitted by the following method: log(inhibitor) vs. response -- Variable slope of GraphPad Prism.

3. **Results and Discussion**

In the cytopathic effect assay, the positive control compound ST-246 showed the expected antiviral activity and cytotoxicity, indicating the reliability of the experiment. The antiviral activity and cytotoxicity results of the test compound and the control compound were summarized in Table 3.

**Table 3. Antiviral Activity Results of Compound**

| No. | Virus | CPD ID | $EC_{50}$ (μM) | $CC_{50}$ (μM) |
|---|---|---|---|---|
| 1 | Vaccinia VP13 | Compound **1** | 0.57 | >10 |
| | | ST-246 | 0.04 | >100 |
| 2 | Monkeypox virus | Compound **1** | 0.32 | >10 |
| | | ST-246 | 0.03 | >100 |
| 3 | Cowpox virus | Compound **1** | 0.75 | >10 |
| | | ST-246 | 0.05 | >100 |
| 4 | Smallpox virus | Compound **1** | 1.2 | >10 |
| | | ST-246 | 0.07 | >100 |

The results showed that Compound **1** had significant inhibitory effects on Vaccinia virus, Monkeypox virus, Cowpox virus, and Smallpox virus, and the $EC_{50}$ values were 0.57, 0.32, 0.75, and 1.20 μM, respectively. While the antiviral activity of Compound **1** was weaker than that of ST-246, Compound **1** has been approved for marketing as a drug for treatment of AIDS and COVID-19, and its safety is guaranteed. Therefore, Compound **1** has greater potential for clinical application.

## Claims

1. Use of a compound represented by formula (I) or a pharmaceutically acceptable salt thereof in a preparation of a drug for preventing or treating an orthopoxvirus infectious disease,

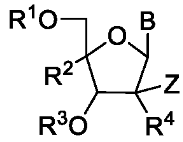

**(I)**

wherein in formula (I),

$R^1$ is H, $R^5$-CO- or

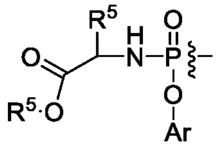

,

wherein Ar is phenyl, substituted phenyl, naphthyl or substituted naphthyl, wherein a substituent is selected from $C_{1-6}$ alkyl, F, Cl, Br, I, CN, $N_3$, OH, $NH_2$, OR $^5$, and $NHR^5$;
$R^2$ is H, azido, $C_1$-$C_6$ alkyl (such as methyl or ethyl), $C_1$-$C_6$ alkoxy (such as methoxy or ethoxy), $C_2$-$C_6$ alkynyl (such as ethynyl), $C_2$-$C_6$ alkenyl (such as vinyl), or halogenated $C_1$-$C_6$ alkyl (such as 2-chloroethyl, 2-fluoroethyl or trifluoroethyl);
$R^3$ is H, optionally substituted R-CO-, optionally substituted R-O(C=O)- or optionally substituted RNH-CO-, wherein R is $C_1$-$C_6$ alkyl (such as methyl, ethyl, propyl, isopropyl, butyl, isobutyl, pentyl or isopentyl), wherein a substituent is selected from $C_1$-$C_6$ alkyl, halogen (such as For Cl), CN, $N_3$, and $OR^5$;
$R^4$ is H, OH, halogen (such as F), $C_1$-$C_6$ alkyl (such as methyl or ethyl) or $C_1$-$C_6$ alkoxy (such as methoxy or ethoxy);

B is selected from:

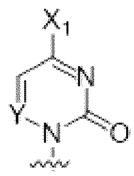

and

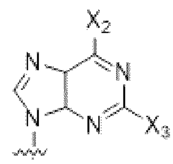

,

wherein $X_1$ is -OH, -$NH_2$, $R^5$CONH-, $R^5$COO- or $R^5$O (C=O)NH-;
$X_2$ is OH, SH, $NH_2$, $R^5$COO-, $R^5$COS-, $R^5$ $CONH_2$- or $R^5$O (C=O)NH-;
$X_3$ is H, F, OH or $NH_2$;
Y is CH or N;
Z is H, OH or F;
$R^5$ is selected from H, $C_1$-$C_6$ alkyl (such as methyl, ethyl, propyl or isopropyl), $C_2$-$C_6$ alkynyl (such as ethynyl), $C_2$-$C_6$ alkenyl (such as vinyl), halogenated $C_1$-$C_6$ alkyl (such as 2-chloroethyl, 2-fluoroethyl or trifluoroethyl), phenyl optionally substituted with $C_{1-6}$ alkyl, $C_{1-6}$ alkoxy, CN, $N_3$, OH, $NH_2$ or halogen (such as F, Cl, Br or I), and naphthyl optionally substituted with $C_{1-6}$ alkyl, $C_{1-6}$ alkoxy, CN, $N_3$, OH, $NH_2$ or halogen (such as F, Cl, Br or I).

2. The use according to claim 1, wherein in formula (I),

$R^1$ is H or $R^5$-CO-;
$R^2$ is azido;
$R^3$ is H or R-CO-, wherein R is $C_1$-$C_6$ alkyl;
$R^4$ is halogen;

B is:

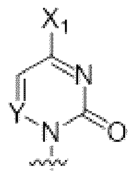,

wherein $X_1$ is $-NH_2$;
Y is CH;
Z is H;
$R^5$ is selected from H and $C_1$-$C_6$ alkyl.

**3.** The use according to claim 1, wherein the compound represented by formula (I) is the following compound:

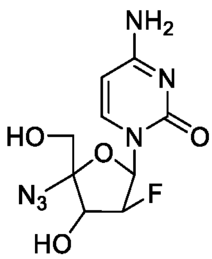,

**1**

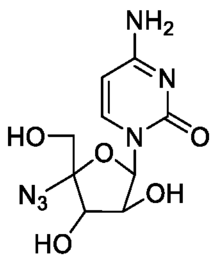,

**2**

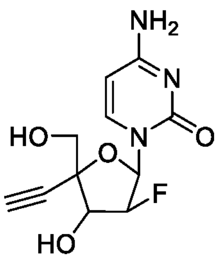,

**3**

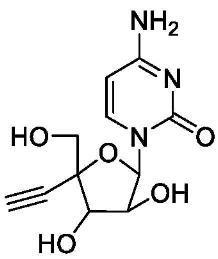,

**4**

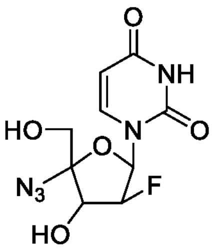,

**5**

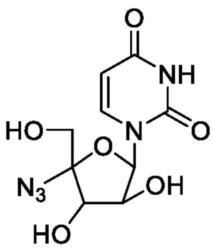,

**6**

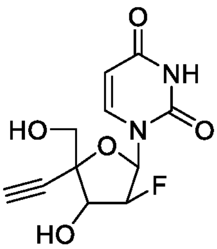,

**7**

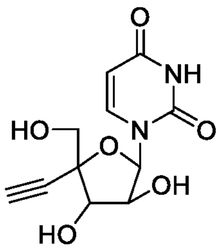

**8**

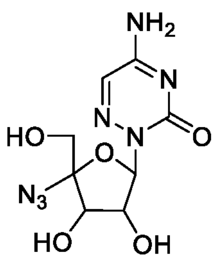,

**9**

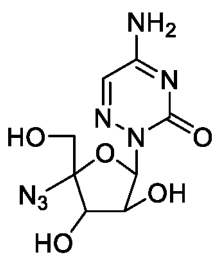,

**10**

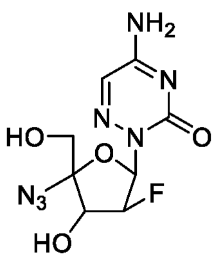,

**11**

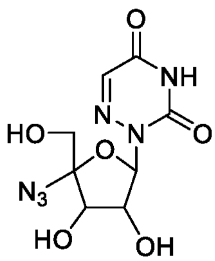

**12**

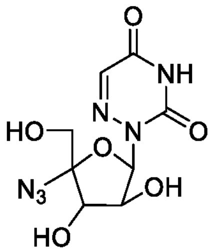,
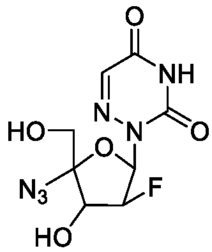,
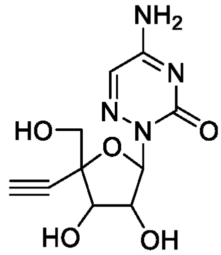,
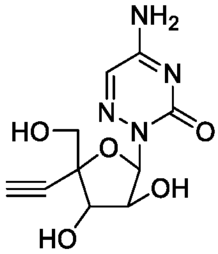

**13** **14** **15** **16**

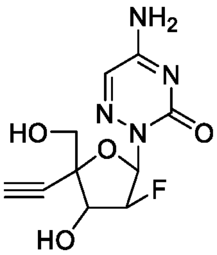,
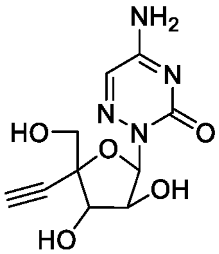,
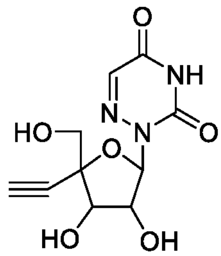,
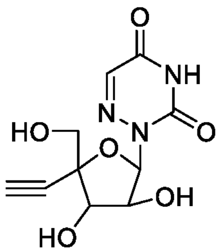

**17** **18** **19** **20**

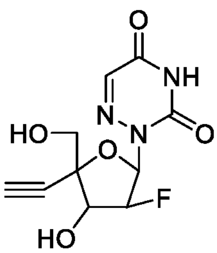,
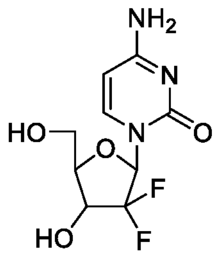,
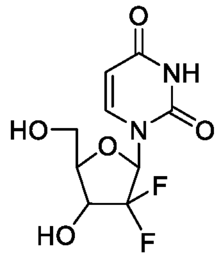,
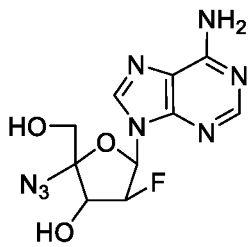,

**21** **22** **23** **24**

or

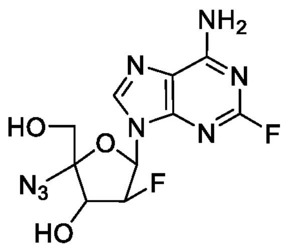.

**25**

4. The use according to any one of claims 1 to 3, wherein the pharmaceutically acceptable salt of the compound represented by formula (I) comprises a salt formed of the compound represented by formula (I) with the following acid: hydrochloric acid, hydrobromic acid, sulfamic acid, sulfuric acid, phosphoric acid, nitric acid, formic acid, acetic acid, propionic acid, oxalic acid, glycolic acid, malonic acid, benzoic acid, lactic acid, gluconic acid, citric acid, tartaric acid, succinic acid, fumaric acid, maleic acid, mandelic acid, malic acid, methanesulfonic acid, ethanesulfonic acid, isethionic acid, benzenesulfonic acid, p-toluenesulfonic acid, naphthalenesulfonic acid, naphthalenedisulfonic acid, camphorsulfonic acid, ascorbyl palmitic acid, salicylic acid, sulfosalicylic acid, 2-hydroxy-3-naphthoic acid, phthalic acid, lysine, arginine, glutamic acid, glycine, serine, threonine, alanine, isoleucine or leucine.

5. The use according to any one of claims 1 to 4, wherein the orthopoxvirus is monkeypox virus, cowpox virus or smallpox virus, and the orthopoxvirus infectious disease includes a disease caused by infection of humans or other animals.

6. The use according to any one of claims 1 to 5, wherein a dosage form of the drug is an immediate-release dosage form, a sustained-release dosage form or a controlled-release dosage form, such as a tablet, a hard or soft capsule, an aqueous or oily suspension, a granule, an emulsion, a syrup, an elixir, an injection or a powder injection.

**INTERNATIONAL SEARCH REPORT**

International application No.
**PCT/CN2023/127028**

**A. CLASSIFICATION OF SUBJECT MATTER**

A61K31/7068(2006.01)i; A61K31/7072(2006.01)i; A61K31/706(2006.01)i; A61K31/7076(2006.01)i; A61P31/20(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, VCN, VEN, CJFD, CNKI, ISI-WEB OF SCIENCE, PUBMED, BING, STNext, 百度学术, BAIDU SCHOLAR, 读秀, DUXIU: 河南真实生物科技有限公司, 王朝阳, 李磐, 贾丽敏, HENAN GENUINE BIOTECH, 核苷类似物, nucleosides, nucleotide, 吉西他滨, 阿兹夫定, RO 0622, 1011529-10-4, 阿糖胞苷, 阿糖腺苷, azvudine, sofosbuvir, 索非布韦, 氮 1W 尿苷, azauridine, cytarabine, 脱氧尿苷, 脱氧胞苷, gemcitabine, 依诺他滨, 痘 1w 病毒, 猴痘, 牛痘, 天花, 痘苗, Orthopoxvirus, Vaccinia, monkeypox, cowpox, smallpox, 结构检索, structural search

**C. DOCUMENTS CONSIDERED TO BE RELEVANT**

| Category* | Citation of document, with indication, where appropriate, of the relevant passages | Relevant to claim No. |
|---|---|---|
| X | WO 03059334 A2 (ELI LILLY AND COMPANY) 24 July 2003 (2003-07-24)<br>description, page 2, line 26 to page 3, line 3, page 4, lines 10-12, and pages 6-10, in vitro results | 1, 3-6 |
| Y | CN 101177442 A (ZHENGZHOU UNIVERSITY et al.) 14 May 2008 (2008-05-14)<br>claims 1-3 and 7 | 1-6 |
| Y | CN 113304166 A (HENAN ZHENSHI BIOTECHNOLOGY CO., LTD.) 27 August 2021 (2021-08-27)<br>claims 1-5 | 1-6 |
| Y | CN 109893536 A (HENAN ZHENSHI BIOTECHNOLOGY CO., LTD.) 18 June 2019 (2019-06-18)<br>claims 1-9 | 1-6 |
| Y | US 2007003516 A1 (ALMOND, M.R. et al.) 04 January 2007 (2007-01-04)<br>claims 1-22, and description, paragraphs 0012-0014 | 1-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 January 2024** | **10 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/127028**

**C. DOCUMENTS CONSIDERED TO BE RELEVANT**

| Category* | Citation of document, with indication, where appropriate, of the relevant passages | Relevant to claim No. |
|---|---|---|
| X | KERN, E.R. "In Vitro Activity of Potential Anti-Poxvirus Agents" *Antiviral Research,* Vol. 57, No. 1, 07 January 2003 (2003-01-07), pages 35-40 | 1, 3-6 |
| Y | KERN, E.R. "In Vitro Activity of Potential Anti-Poxvirus Agents" *Antiviral Research,* Vol. 57, No. 1, 07 January 2003 (2003-01-07), pages 35-40 | 1-6 |
| X | BALBONI, P. G. et al. "Activity of Albumin Conjugates of 5-Fluorodeoxyuridine And Cytosine Arabinoside on Poxviruses as a Lysosomotropic Approach to Antiviral Chemotherapy" *Nature,* Vol. 264, 11 November 1976 (1976-11-11), pages 181-183 | 1, 4-6 |
| X | SMEE, D.F. et al. "Comparison of Three Dimensional Synergistic Analyses of Percentage Versus Logarithmic Data in Antiviral Studies" *Antiviral Research,* 01 July 2017 (2017-07-01), pages 1-12 | 1, 4-6 |

Form PCT/ISA/210 (second sheet) (July 2022)

**INTERNATIONAL SEARCH REPORT**
**Information on patent family members**

International application No.
**PCT/CN2023/127028**

| Patent document cited in search report | Publication date (day/month/year) | Patent family member(s) | Publication date (day/month/year) |
|---|---|---|---|
| WO 03059334 A2 | 24 July 2003 | WO 03059334 A3 | 30 October 2003 |
| | | AU 2002365256 A1 | 30 July 2003 |
| CN 101177442 A | 14 May 2008 | US 2010234584 A1 | 16 September 2010 |
| | | US 8835615 B2 | 16 September 2014 |
| | | KR 20100102089 A | 20 September 2010 |
| | | JP 2010533659 A | 28 October 2010 |
| | | EP 2177527 A1 | 21 April 2010 |
| | | EP 2177527 A4 | 14 July 2010 |
| | | EP 2177527 B1 | 18 December 2013 |
| | | WO 2009009951 A1 | 22 January 2009 |
| CN 113304166 A | 27 August 2021 | None | |
| CN 109893536 A | 18 June 2019 | WO 2020007070 A1 | 09 January 2020 |
| | | US 2023151048 A1 | 18 May 2023 |
| US 2007003516 A1 | 04 January 2007 | HK 1113085 A1 | 26 September 2008 |
| | | WO 2006110655 A2 | 19 October 2006 |
| | | WO 2006110655 A3 | 28 June 2007 |
| | | JP 2013231058 A | 14 November 2013 |
| | | EP 1868628 A2 | 26 December 2007 |
| | | EP 1868628 A4 | 02 December 2009 |
| | | EP 1868628 B1 | 11 June 2014 |
| | | JP 2008535862 A | 04 September 2008 |
| | | US 8642577 B2 | 04 February 2014 |

Form PCT/ISA/210 (patent family annex) (July 2022)